# EUROPEAN PATENT APPLICATION

(11) **EP 4 385 540 A1**
(43) Date of publication of application: **19.06.2024**
(21) Application number: 22882336.5
(22) Date of filing: 29.06.2022
(51) Int. Cl.: A61M 5/158

(54) **VERTICAL INDWELLING NEEDLE ASSEMBLY AND BASE STRUCTURE THEREOF**

(30) Priority: 19.10.2021 CN 202111214497
(71) Applicant: Aaruy Medical (Guangdong) Co., Ltd., Dongguan, Guangdong 523000 (CN)
(72) Inventor: ZHOU, Qingxu, Dongguan, Guangdong 523000 (CN)
(74) Representative: Petraz, Gilberto Luigi
(86) International application number: PCT/CN2022/102229
(87) International publication number: WO 2023/065713

(57) **Abstract**

Disclosed in the present application is a vertical indwelling needle assembly, including a base body, an interior of the base body is provided with an infusion chamber, a straight channel and an infusion channel; the transverse channel is provided proximal to a bottom of the base body; the connecting channel is used to communicate the infusion chamber with the transverse channel; the transverse channel extends transversely to an exterior surface of the base body to form an external opening; an interior of the transverse channel is provided with a second flexible sealing member; the second flexible sealing member is provided between a connecting opening and the external opening. Thus, the entire strength of the base structure is effectively enhanced to ensure the reliability of the vertical indwelling needle assembly; in addition, since complicated channel structures are unnecessary to be disposed on the connector cooperating with the base structure mentioned above, it is sufficient to only design the infusion needle, so as to greatly simplify the structure of the connector and contribute to the improvement of the production efficiency of the entire vertical indwelling needle assembly.

## Description

### Field of the invention

The present application relates to the technical field of medical instruments, in particular to a vertical indwelling needle assembly and a base structure thereof.

### Background of the invention

In the prior art, the indwelling needle of the infusion pumps (e.g., insulin pumps) is divided into two types, i.e., the flexible indwelling needle and the steel indwelling needle, in which the steel indwelling needle is harmful to the human body, easily causing fear to the patient and are not well accepted. Therefore, the flexible indwelling needle are currently more common on the market.

In view of a vertical indwelling needle, the vertical indwelling needle belongs to the flexible indwelling needle and includes a base structure and a connector, in which the base structure is formed with an infusion chamber. A bottom of the base structure is formed with a flexible indwelling infusion needle communicating with the infusion chamber. The connector is used to connect to the base structure, so as to allow the medicinal liquid within the reservoir of the infusion pump to be conveyed through the connector to the infusion chamber.

Generally, the more proximal the infusion tube of the connector is to a patient's skin surface, the better the patient's experience is. However, in order to satisfy the patient's experience, regarding the current vertical indwelling needle, either the infusion chamber inside the base structure occupies too much volume, causing the base structure prone to crack due to insufficient strength of the base structure, which deteriorates the reliability of the vertical needle assembly, or the structure of the connector is relatively complex, which results in the low production efficiency of the entire vertical indwelling needle assembly.

In summary, how to solve the problems of poor reliability and low production efficiency of the vertical indwelling needle assembly has become an urgent technical problem to be solved by those skilled in the art.

### Summary of the invention

An objective of the present application is to provide a vertical indwelling needle assembly and a base structure thereof, so as to solve the problems of low reliability and complicated production process of the vertical indwelling needle assembly.

In order to achieve the objective mentioned above, provided in the present application is a base structure of a vertical indwelling needle, including a base body for detachably connected to a connector of the vertical indwelling needle. An interior of the base body is provided with an infusion chamber, a straight channel and an infusion channel; the infusion chamber is mounted proximal to a top of the base body; a bottom of the base body is provided with a flexible indwelling infusion needle mounted vertically to a bottom surface of the base body; a top of the straight channel is in communication with the infusion chamber; a bottom of the straight channel is coaxially in communication with a starting end of the flexible indwelling infusion needle; an upper chamber opening of the infusion chamber is provided with a first flexible sealing member for sealing the upper chamber opening; the first flexible sealing member is used to allow a penetration of a puncture needle of an insertion device, so as to allow the puncture needle to pass through the infusion chamber and the straight channel and to extend out of an end of the flexible indwelling infusion needle; the infusion channel includes a transverse channel and a connecting channel both formed in the base body; the transverse channel is provided proximal to a bottom of the base body; the connecting channel is used to communicate the infusion chamber with the transverse channel; the transverse channel extends transversely to an exterior surface of the base body to form an external opening; an interior of the transverse channel is provided with a second flexible sealing member; the second flexible sealing member is provided between a connecting opening and the external opening, in which the connecting opening is positioned on the transverse channel correspondingly communicating with the connecting channel; and the infusion needle of the connector penetrates the second flexible sealing member from the external opening and is in communication with an interior of the transverse channel when the connector is assembled in place with the base body.

Preferably, the infusion chamber is a funnel-shaped structure disposed along an assembly axis of the base body; a top of the funnel-shaped structure forms a wide opening; the wide opening forms the upper chamber opening; a bottom of the funnel-shaped structure forms a leak opening; and an upper opening of the straight channel is connected to the leak opening.

Preferably, the base body is provided with a groove connecting to the wide opening at a position corresponding to an upper position of the wide opening, and the first flexible sealing member is embedded in the groove.

Preferably, a top of the base body is further provided with an upper cover for restricting the first flexible sealing member within the groove, and a top of the upper cover is provided with an avoidance hole for passing through the puncture needle.

Preferably, the upper cover is provided on a top of the base body by ultrasonic welding.

Preferably, the connecting channel includes a first connecting channel disposed horizontally and a second connecting channel disposed vertically; an end of the first connecting channel is in communication with the infusion chamber, while an opposite end of the first connecting channel is in communication with an end of the second connecting channel; an opposite end of the second connecting channel is in communication with the transverse channel; and the second flexible sealing member is positioned between the connecting opening and the external opening, in which the connecting opening is positioned on the transverse channel correspondingly communicating with the second connecting channel.

Preferably, a lateral part of the base body is provided with a second snap-fit part for snap-fitting to a first snap-fit part of the connector, and the first snap-fit part is snap-fitted with the second snap-fit part in place when the connector is assembled in place with the base body.

Compared to the introduced background description, adopting the base structure of the vertical indwelling needle assembly during a practical application, as the base structure is internally designed with an infusion channel communicating with a infusion chamber, and the infusion channel includes a transverse channel and a connecting channel, in which the infusion needle of the connector may puncture the second flexible sealing member from an external opening of the transverse channel and connect to an interior of the transverse channel. Despite that the infusion chamber is positioned on a top of the base body, however, the presence of the connecting channel allows the transverse channel to still be disposed proximal to a bottom of the base body, so that the infusion tube connected to the infusion needle on the connector is more proximal to patient's skin surface and the patient's experience is improved. Also, since the infusion chamber is unnecessary to be designed to directly in communication with the transverse channel in a transverse direction, the longitudinal depth of the infusion chamber may be effectively reduced with a certain overall height of the base structure, which is conducive to reducing the occupancy volume of the infusion chamber, improving the overall strength of the base structure, avoiding the problem that the base structure is prone to cracking, and ensuring the reliability of the vertical needle assembly. In addition, since a complicated channel structure is unnecessary to be provided on the connector cooperating with the base structure, it is sufficient to only design the infusion needle, so as to simplify the structure of the connector and contribute to the improvement of the production efficiency of the entire vertical indwelling needle assembly.

In addition, provided in the present application is a vertical indwelling needle assembly, including a base structure and a connector detachably connected to the base structure, the base structure being the base structure of the vertical indwelling needle assembly described in any one of the above implementations. Since the base structure mentioned before provides the technical effects mentioned above, thus the vertical indwelling needle assembly provided with the base structure should also provide the corresponding technical effects, which is not repeated herein.

Preferably, the vertical indwelling needle assembly further includes a protective cover, and the protective cover detachably covers on the connector or the base structure.

Preferably, the vertical indwelling needle assembly further includes an insertion device, the insertion device including a cover shell and a puncture needle provided in the cover shell; the base structure is provided with a puncture needle holder for cooperating with the cover shell; and the puncture needle extends out of an end of the flexible indwelling infusion needle when the cover shell fits in place with the puncture needle holder.

### Brief description of the drawings

Fig. 1 is a structural diagram in a perspective view of the vertical indwelling needle assembly of an embodiment in the present application;
Fig. 2 is a structural diagram in a top view of the vertical indwelling needle assembly of an embodiment in the present application;
Fig. 3 is a structural diagram in a cross-sectional view of the Fig. 2 taken along line A-A;
Fig. 4 is a structural diagram in a perspective view of the base structure of an embodiment in the present application;
Fig. 5 is a structural diagram in a top view of the base structure of an embodiment in the present application;
Fig. 6 is a structural diagram in a cross-sectional view of the Fig. 5 taken along line B-B;
Fig. 7 is a structural diagram in a perspective view of the connector of an embodiment in the present application;
Fig. 8 is a structural diagram in a top view of the connector of an embodiment in the present application;
Fig. 9 is a structural diagram in a cross-sectional view of the Fig. 8 taken along line C-C.

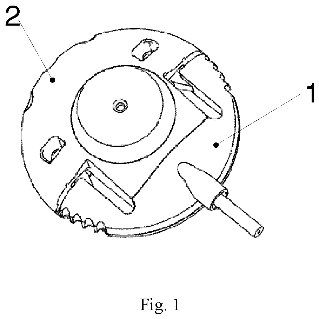

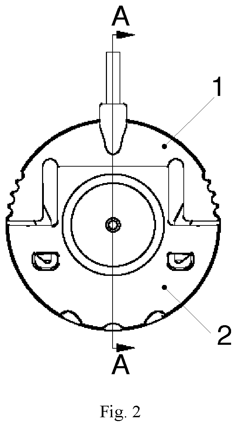

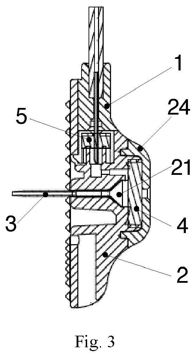

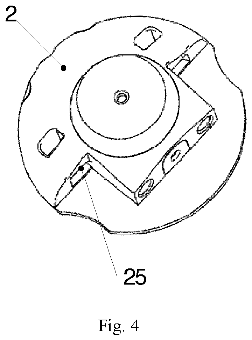

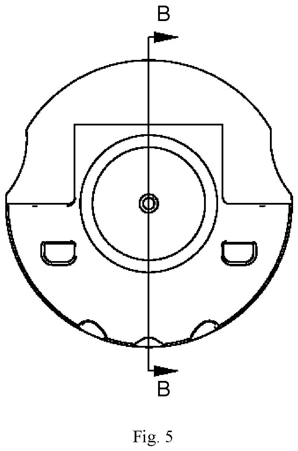

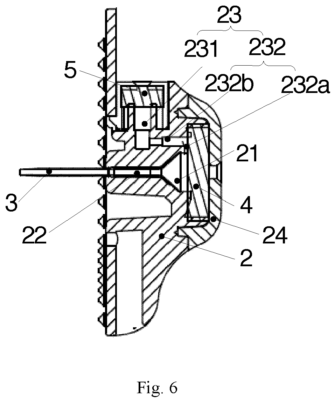

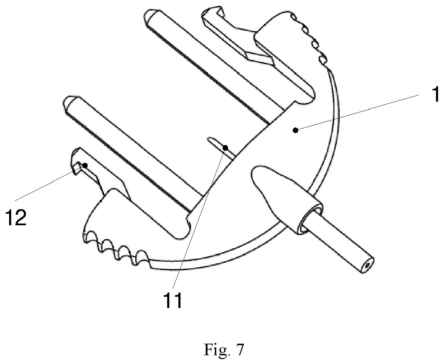

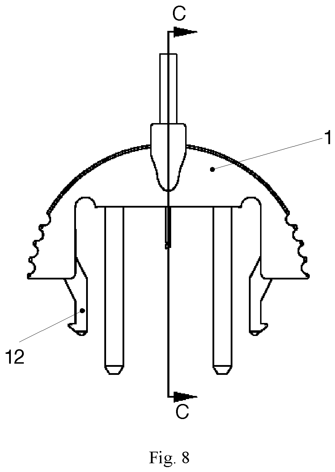

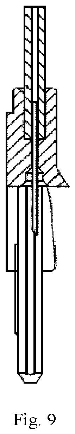

In Figs. 1-9,
1 connector; 11 infusion needle; 12 first snap-fit part; 2 base body; 21 infusion chamber; 22 straight channel; 23 infusion channel; 231 transverse channel; 232 connecting channel; 232a first connecting channel; 232b second connecting channel; 24 upper cover; 25 second snap-fit part; 3 flexible indwelling infusion needle; 4 first flexible sealing member; 5 second flexible sealing member.

### Detailed description of the preferred embodiments

The core of the present application is to provide a vertical indwelling needle assembly and a base structure thereof, so as to solve the problems of low reliability and complicated production process of the vertical indwelling needle assembly.

For a better understanding of the provided solutions in the present application by those skilled in the art, the technical solutions in the examples of the present application are clearly and completely described and discussed below in conjunction with the attached drawings of the embodiments of the present application. Obviously, the examples described herein are only some of the examples of the present application but not all of them. Based on the embodiments in the present application, all other embodiments obtained by those skilled in the art without creative efforts fall within the scope of protection of the present application.

Referring to Figs. 1-9, provided in an embodiment of the present application is a base structure of a vertical indwelling needle, including a base body 2 used for detachably connecting to a connector 1. An interior of the base body 2 is provided with an infusion chamber 21, a straight channel 22 and an infusion channel 23; the infusion chamber 21 is mounted proximal to a top of the base body 2; a bottom of the base body 2 is provided with a flexible indwelling infusion needle 3 mounted vertically to a bottom surface of the base body 2; a top of the straight channel 22 is in communication with the infusion chamber 21; a bottom of the straight channel 22 is coaxially in communication with a starting end of the flexible indwelling infusion needle 3; an upper chamber opening of the infusion chamber 21 is provided with a first flexible sealing member 4 for sealing the upper chamber opening; the first flexible sealing member 4 is used to allow a penetration of a puncture needle of an insertion device, so as to allow the puncture needle to pass through the infusion chamber 21 and the straight channel 22 and to extend out of an end of the flexible indwelling infusion needle 3; the infusion channel 23 includes a transverse channel 231 and a connecting channel 232 both formed in the base body 2; the transverse channel 231 is provided proximal to a bottom of the base body 2; the connecting channel 232 is used to communicate the infusion chamber 21 with the transverse channel 231; the transverse channel 231 extends transversely to an exterior surface of the base body 2 to form an external opening; an interior of the transverse channel 231 is provided with a second flexible sealing member 5; the second flexible sealing member 5 is provided between a connecting opening and the external opening, in which the connecting opening is positioned on the transverse channel 231 correspondingly communicating with the connecting channel 232; and the infusion needle 11 of the connector 1 penetrates the second flexible sealing member 5 from the external opening and is in communication with an interior the transverse channel 231 when the connector 1 is assembled in place with the base body 2.

During a practical application, by adopting the base structure of the vertical indwelling needle assembly, since an interior of the base structure is designed to be provided with an infusion channel communicating with an infusion chamber and the infusion channel includes both a transverse channel and a connecting channel, the infusion needle of the connector may puncture the second flexible sealing member from an external opening of the transverse channel and is in communication with an interior of the transverse channel. Despite that the infusion chamber is positioned on a top of the base body, the presence of the connecting channel allows the transverse channel to still be disposed proximal to a bottom of the base body, so that the infusion tube connected to the infusion needle on the connector is more proximal to patient's skin surface and the patient's experience is improved. Also, since the infusion chamber is unnecessary to be designed to be directly in communication with the transverse channel in a transverse direction, the longitudinal depth of the infusion chamber may be effectively reduced with a certain overall height of the base structure, which is conducive to reducing the occupancy volume of the infusion chamber, improving the overall strength of the base structure, avoiding the problem that the base structure is prone to cracking, and ensuring the reliability of the vertical indwelling needle assembly. In addition, since a complicated channel structure is unnecessary to be provided on the connector cooperating with the base structure, it is sufficient to only design the infusion needle, so as to simplify the structure of the connector and contribute to the improvement of the production efficiency of the entire vertical indwelling needle assembly. Additionally, the connector is detachably connected to a lateral part of the base body, which is easier to operate.

For a better understanding of the technical solution of the present invention by those skilled in the art, a brief description of the using process of the base structure mentioned above is as follows.

During a practical application, the bottom surface of the base body 2 is firstly attached to the patient's skin surface. Then the base structure assembling in place with the connector 1 is cooperated with the insertion device. The puncture needle provided in the insertion device passes through the first flexible sealing member 4, the infusion chamber 21 and the straight channel 22 and extends out of an end of the flexible indwelling infusion needle 3, so that the puncture needle assists in delivering the flexible indwelling infusion needle 3 into the patient's skin. Then the insertion device is removed, that is, the fixation of the base structure to the patient is completed. In such a case, during the operation, a subcutaneous area may be isolated from outside air due to the presence of the first flexible sealing member. The reservoir of the infusion pump input the medicinal liquid from an inlet connection transversely disposed on a lateral part of the connector by an infusion tube. The medicinal liquid passes through the infusion needle (generally a rigid projecting needle) of the connector which has penetrated the second flexible sealing member, and is conveyed via the transverse channel to the connecting channel, and then arrives the infusion chamber disposed vertically through the connecting channel, so that the medicinal liquid is conveyed into the subcutaneous area by the flexible indwelling infusion needle.

It is to be noted that the infusion channel 23 may be specifically formed by integral casting with the base body 2. Admittedly, other methods known to those skilled in the art may also be adopted during actual application, such as embedding hardware to an interior of the base body. However, with such an arrangement, the complicated production process of embedding hardware to an interior of the base body may be avoided, which reduces the production difficulty and also improves the reliability.

It is also to be noted that the transverse, vertical, horizontal and longitudinal directions mentioned above are definitions of orientation based on the bottom surface of the base body. Specifically, transverse and horizontal refer to directions parallel to the bottom surface of the base body, and vertical and longitudinal refer to directions vertical to the bottom surface of the base body. In general, the bottom surface of the base body is attached to the patient's skin surface.

In addition, the first/second flexible sealing member mentioned above may specifically be a rubber plug, or other flexible sealing members known to those skilled in the art, which is not more specifically limited herein. The connector and the reservoir of the infusion pump mentioned above are specifically in communication through the infusion tube, in which a flexible catheter is generally adopted as the infusion tube. However, the connection manner between the infusion tube and the connector 1 may adopt a detachable connection, such as that the connector 1 is provided with a connection where the infusion tube is sleeved thereon, or adopt an embedded fixing connection, which is not specifically limited herein. The bottom surface of the base body is generally designed as a plane, primarily to better fit the patient's skin surface. Generally, a periphery of the bottom surface of the base body corresponding to the flexible indwelling infusion needle is further provided with an annular groove. The role of the annular groove is mainly that, when the bottom surface of the base is in contact with the patient's skin, part of the skin falls into the annular groove due to the elasticity of the skin surface layer, resulting in a good barrier effect on the wound position where the soft indwelling infusion needle penetrates into the skin, reducing the risk of wound infection.

In some specific implementations, the infusion chamber 21 is a funnel-shaped structure disposed along an assembly axis of the base body 2; a top of the funnel-shaped structure forms a wide opening; the wide opening forms the upper chamber opening; a bottom of the funnel-shaped structure forms a leak opening; and an upper opening of the straight channel 22 is connected to the leak opening. By designing the infusion chamber 21 as a funnel-shaped structure, the medicinal liquid may enter the straight channel 22 and the flexible indwelling infusion needle 3 more smoothly. Admittedly, it is to be understood that the funnel-shaped structure of the infusion chamber is only a preferred example of an embodiment of the present application. Other structural forms may also be adopted during the actual application, such as hemispherical chambers, which is not more specifically limited herein.

In a further implementation, the base body 2 is provided with a groove connecting to the wide opening at a position corresponding to an upper position of the wide opening, and the first flexible sealing member 4 is embedded in the groove. By providing the groove, it is much easier to install and fix the first flexible sealing member. Admittedly, it is to be understood that the method of fixing the flexible sealing member by embedding it in the groove is only a preferred example of an embodiment of the present application. There may be other fixing methods commonly used by those skilled in the art during actual application, such as a bonding fixing, which is not more specifically limited herein.

In some specific implementations, a top of the base body 2 is further provided with an upper cover 24 for restricting the first flexible sealing member 4 within the groove, and a top of the upper cover 24 is provided with an avoidance hole 241 for passing through the puncture needle. By providing the upper cover structure, it is more convenient to install, fix as well as disassemble the first flexible sealing member.

It is to be noted that the provision of the upper cover on the base body may adopt a detachable connection, such as a snap-fit or threaded connection, or a fixed connection, such as the upper cover being provided on a top of the base body 2 by ultrasonic welding.

In some specific implementations, the connecting channel 232 includes a first connecting channel 232a disposed horizontally and a second connecting channel 232b disposed vertically; an end of the first connecting channel 232a is in communication with the infusion chamber 21, while an opposite end of the first connecting channel 232a is in communication with an end of the second connecting channel 232b; an opposite end of the second connecting channel 232b is in communication with the transverse channel 231; and the second flexible sealing member 5 is positioned between the connecting opening and the external opening, in which the connecting opening is positioned on the transverse channel 231 correspondingly communicating with the second connecting channel 232b. It is more convenient to process the connecting channel by designing the connecting channel into a structural manner of the first connecting channel 232a disposed horizontally and the second connecting channel 232b disposed vertically. Admittedly, it is to be understood that the connecting channel may further be designed into a structural manner of a curved channel or a special-shaped channel during a practical application, however, which is more complicated to process.

In some further specific implementations, a lateral part of the base body 2 is provided with a second snap-fit part 25 for snap-fitting to a first snap-fit part 12 of the connector 1, and the first snap-fit part 12 is snap-fitted with the second snap-fit part 25 in place when the connector 1 is assembled in place with the base body 2. It is more convenient to achieve the disassembly and assembly by designing that the first snap-fit part 12 and the second snap-fit part 25 are connected by a snap fit. Admittedly, other detachable connection methods commonly used by those skilled in the art may be adopted during a practical application, such as the method of a threaded connection. When adopting the snap-fit connection method, the first snap-fit part 12 may specifically be a tongue provided in the connector, and the second snap-fit part 25 may specifically be a slot formed on the base body 2. The design may certainly be reversed. For example, the first snap-fit part 12 may specifically be a slot provided in the connector, and the second snap-fit part 25 may specifically be a tongue formed on the base body 2. It may be selected according to the actual demand during a practical application, which is not more specifically limited herein.

In addition, provided in the present application is a vertical indwelling needle assembly, including a base structure and a connector detachably connected to the base structure, in which the base structure is the base structure of the vertical indwelling needle assembly described in any one of the above solutions. Since the base structure mentioned before provides the technical effects mentioned above, the vertical indwelling needle assembly provided with the base structure should also provide the corresponding technical effects, which is not repeated herein.

It is noted that the vertical indwelling needle assembly is primarily applied to the delivery of medicinal liquid to subcutaneous tissues, such as the infusion of insulin or other medicinal liquid requiring precise control of dosage, which is not more specifically limited herein. Taking the application to insulin infusion for an example, the vertical indwelling needle assembly is generally required to remain mounted on the patient for a sustained period of time, which may specifically be several days, such as 3-5 days or even a week or more. However, it is inevitable that the patient may be exposed to water during this period of time, such as bathing. In such a case, the vertical indwelling needle assembly is required to be briefly detached from the infusion pump system. Therefore, a better protection of the vertical indwelling needle assembly is then required to avoid infection of the needle puncture wound.

In order to avoid the risk of infection due to being exposed to water, the vertical indwelling needle assembly may also include a protective cover, and the protective cover detachably covers on the connector 1 or the base structure 2. When the patient requires an exposure to water, such as bathing, the connector 1 may simply be detached while the protective cover is mounted to the base structure 2 to achieve the sealing.

In some more specific implementations, the vertical indwelling needle assembly further includes an insertion device, the insertion device comprises a cover shell and a puncture needle provided in the cover shell; the base structure is provided with a puncture needle holder for cooperating with the cover shell; and the puncture needle extends out of an end of the flexible indwelling infusion needle when the cover shell fits in place with the puncture needle holder. By cooperating the cover shell with the base, it is more convenient and accurate to match the puncture needle with the flexible indwelling infusion needle.

The vertical indwelling needle assembly and the base structure thereof provided by the present application are described in detail above. It should be noted that the various embodiments in the present specification are described in a progressive manner. Each embodiment focuses on the differences with other embodiments. It is sufficient to refer to each embodiment for the same and similar parts.

It should also be noted that, as used herein, the related terms such as "include", "comprise", or any other derivatives and conjugations thereof, are intended to cover non-exclusive inclusion. Thus, an object or apparatus including a series of elements includes not only those elements, but also other elements that are not explicitly listed, or that are inherent to such an object or apparatus. In the absence of further limitation, an element defined by the phrase "including a/an ..." does not exclude the existence of another identical element in the object or apparatus including the aforementioned element.

Specific embodiments are used herein to illustrate the principles and implementations of the present application, and the above illustrations of the embodiments are only used to assist in the understanding of the core principles of the present application. It should be noted that for those skilled in the art, a plurality of improvements and modifications may be made without departing from the principles of the present application, which also fall within the scope of protection of the claims of the present application.

## Claims

1. A base structure of a vertical indwelling needle assembly, comprising: a base body (2) used for detachably connecting to a connector (1) of the vertical indwelling needle assembly, wherein an interior of the base body (2) is provided with an infusion chamber (21), a straight channel (22) and an infusion channel (23);
the infusion chamber (21) is mounted proximal to a top of the base body (2); a bottom of the base body (2) is provided with a flexible indwelling infusion needle (3) mounted vertically to a bottom surface of the base body (2); a top of the straight channel (22) is in communication with the infusion chamber (21); a bottom of the straight channel (22) is coaxially in communication with a starting end of the flexible indwelling infusion needle (3); an upper chamber opening of the infusion chamber (21) is provided with a first flexible sealing member (4) for sealing the upper chamber opening; the first flexible sealing member (4) is used to allow a penetration of a puncture needle of an insertion device, so as to allow the puncture needle to pass through the infusion chamber (21) and the straight channel (22) and to extend out of an end of the flexible indwelling infusion needle (3);
the infusion channel (23) comprises a transverse channel (231) and a connecting channel (232) both formed in the base body (2); the transverse channel (231) is provided proximal to a bottom of the base body (2); the connecting channel (232) is used to communicate the infusion chamber (21) with the transverse channel (231); the transverse channel (231) extends transversely to an exterior surface of the base body (2) to form an external opening; an interior of the transverse channel is provided with a second flexible sealing member (5); the second flexible sealing member (5) is provided between a connecting opening and the external opening, wherein the connecting opening is positioned on the transverse channel (231) correspondingly communicating with the connecting channel (232); and the infusion needle (11) of the connector (1) penetrates the second flexible sealing member (5) from the external opening and is in communication with an interior of the transverse channel (231) when the connector (1) is assembled in place with the base body (2).

2. The base structure of the vertical indwelling needle assembly according to claim 1, wherein the infusion chamber (21) is a funnel-shaped structure disposed along an assembly axis of the base body (2); a top of the funnel-shaped structure forms a wide opening; the wide opening forms the upper chamber opening; a bottom of the funnel-shaped structure forms a leak opening; and an upper opening of the straight channel (22) is connected to the leak opening.

3. The base structure of the vertical indwelling needle assembly according to claim 2, wherein the base body (2) is provided with a groove connecting to the wide opening at a position corresponding to an upper position of the wide opening, and the first flexible sealing member (4) is embedded in the groove.

4. The base structure of the vertical indwelling needle assembly according to claim 3, wherein a top of the base body (2) is further provided with an upper cover (24) for restricting the first flexible sealing member (4) within the groove, and a top of the upper cover (24) is provided with an avoidance hole (241) for passing through the puncture needle.

5. The base structure of the vertical indwelling needle assembly according to claim 4, wherein the upper cover (24) is provided on a top of the base body (2) by ultrasonic welding.

6. The base structure of the vertical indwelling needle assembly according to claim 1, wherein the connecting channel (232) comprises a first connecting channel (232a) disposed horizontally and a second connecting channel (232b) disposed vertically; an end of the first connecting channel (232a) is in communication with the infusion chamber (21), while an opposite end of the first connecting channel (232a) is in communication with an end of the second connecting channel (232b); an opposite end of the second connecting channel (232b) is in communication with the transverse channel (231); and the second flexible sealing member (5) is positioned between the connecting opening and the external opening, wherein the connecting opening is positioned on the transverse channel (231) correspondingly communicating with the second connecting channel (232b).

7. The base structure of the vertical indwelling needle assembly according to any one of claims 1-6 , wherein a lateral part of the base body (2) is provided with a second snap-fit part (25) for snap-fitting to a first snap-fit part (12) of the connector (1), and the first snap-fit part (12) is snap-fitted with the second snap-fit part (25) in place when the connector (1) is assembled in place with the base body (2).

8. A vertical indwelling needle assembly, comprising a base structure and a connector detachably connected to the base structure, wherein the base structure is the base structure of the vertical indwelling needle assembly as claimed in any one of claims 1-7.

9. The vertical indwelling needle assembly according to claim 8, further comprising a protective cover, and the protective cover detachably covers on the connector (1) or the base structure (2).

10. The vertical indwelling needle assembly according to claim 8, further comprising an insertion device, wherein the insertion device comprises a cover shell and a puncture needle provided in the cover shell; the base structure is provided with a puncture needle holder for cooperating with the cover shell; and the puncture needle extends out of an end of the flexible indwelling infusion needle (3) when the cover shell fits in place with the puncture needle holder.
